# EUROPÄISCHE PATENTSCHRIFT

(11) **EP 3 253 361 B1**
(45) Veröffentlichungstag und Bekanntmachung des Hinweises auf die Patenterteilung: **04.12.2019**
(21) Anmeldenummer: 15787214.4
(22) Anmeldetag: 30.10.2015
(51) Int. Cl.: A61K 8/31, A61Q 5/08, A61K 8/73, A61K 8/23

(54) **BLONDIERMITTEL MIT REDUZIERTER ÖLABSCHEIDUNG**
BLEACHING AGENTS HAVING REDUCED OIL-SEPARATION TENDENCY
AGENTS ÉCLAIRCISSANTS À SÉPARATION D'HUILE RÉDUITE

(30) Priorität: 06.02.2015 DE 102015202189
(43) Veröffentlichungstag der Anmeldung: 13.12.2017
(73) Patentinhaber: Henkel AG & Co. KGaA, 40589 Düsseldorf (DE)
(72) Erfinder: ERKENS, Udo, 47877 Willich (DE); MÜLLER, Burkhard, 40221 Düsseldorf (DE)
(86) Internationale Anmeldenummer: PCT/EP2015/075201
(87) Internationale Veröffentlichungsnummer: WO 2016/124264

(56) Entgegenhaltungen:
- EP-A1- 1 034 777
- EP-A2- 0 882 444
- DE-A1-102008 017 439
- JP-A- 2002 302 427
- US-B2- 6 703 004

## Beschreibung

Die vorliegende Erfindung betrifft Mittel zur oxidativen Farbveränderung im Bereich der Kosmetik, die sich besonders zum Aufhellen keratinischer Fasern, insbesondere menschlicher Haare, eignen.

Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Aus Stabilitätsgründen werden handelsübliche Blondiermittel gewöhnlich in zwei getrennt voneinander verpackten Zubereitungen angeboten, die unmittelbar vor der Anwendung zu einer fertigen Anwendungszubereitung vermischt werden. Üblicherweise bestehen handelsübliche Blondiermittel aus einer flüssigen Oxidationsmittelzubereitung und einem Pulver, das feste Oxidationsmittel enthält. Alternativ können anstelle des Pulvers pastenförmige Mittel mit einer flüssigen Oxidationsmittelzubereitung vermischt werden, wodurch die Problematik des Staubens bei der Herstellung und beim Vermischen vermieden wird. Produkte mit weiteren Komponenten werden ebenfalls im Handel angeboten.

Pastenförmige Blondiermittel enthalten meist größere Mengen eines inerten Öls, was zu Stabilitätsproblemen (Absetzen der festen Oxidationsmittel vom Öl sowie Abscheidung der Ölkomponente) führen kann. Selbst bei noch nicht vollständig abgesetzten Peroxidisulfaten kann ein Konzentrationssgradient innerhalb der Verpackung auftreten, so daß verschiedene Portionen aus der Verpackung nach dem Vermischen unterschiedliche Aufhellung bewirken können. Um diese Probleme zu minimieren, ist eine hohe Viskosität wünschenswert.

Auf der anderen Seite muß die Viskosität der Blondierpaste so gering sein, daß sie sich gut und schnell mit der flüssigen Oxidationsmittelzubereitung vermischen läßt. Im Falle wasserfreier Blondiermittel ist die leichte und homogene Mischbarkeit mit der zumeist wäßrigen Oxidationsmittelzubereitung ein besonders wichtiges und technisch aufwendig zu realisierendes Erfordernis.

Die resultierende Blondiermischung muß darüber hinaus flüssig genug sein, um sich leicht und gleichmäßig applizieren zu lassen, aber dickflüssig genug, um nicht vom Kopf oder von Anwendungshilfsmitteln wie Pinseln herabzutropfen. Zusätzlich sollte sich auch die resultierende Blondiermischung nicht trennen, da Absetzen oder Phasenseparationen vom Kunden als Qualitätsmangel wahrgenommen werden.

Oft sollen Haare nicht nur aufgehellt, sondern gleichzeitig gefärbt werden. Blondierpasten sollten daher auch mit cremeförmigen Färbemitteln gut mischbar sein. Oft enthalten diese Färbemittel direktziehende Farbstoffe, so daß eine homogene Vermischung für ein gleichmäßiges Färbe- und Aufhellergebnis eine Voraussetzung ist.

Die WO 2009/134875 A1 beschreibt Blondiermittel, welche Persulfatsalze und ein Ölgel enthalten, das seinerseits aus Öl(en) und bestimmten Polymeren zusammengesetzt ist.

Als wünschenswerte Eigenschaften des Mittels gemäß dieser Erfindung sind Stabilität gegen Absetzen und Phasentrennung angegeben.

Die EP 1 034 777 A1 offenbart Mittel zum Aufhellen keratinischer Fasern, welche mindestens zwei getrennt voneinander verpackte Zubereitungen (A) und (B) enthalten, die unmittelbar vor der Anwendung zu einer Anwendungsmischung vermischt werden, wobei die Zubereitungen (A) ölbasiert sind und Polymer(e) enthalten, welche Oleo- bzw. Lipogele ausbilden. Dabei werden auch Mischungen mit natürlichen Polymeren, beispielsweise Xanthan, offenbart.

Aufgabe der vorliegenden Erfindung war es, die Eigenschaften von Blondiermitteln weiter zu verbessern. dabei sollte insbesondere die Stabilität gegen Ölabscheidung erhöht werden. Darüber hinaus sollte die Mischbarkeit mit farbstoffhaltigen Formulierungen verbessert werden.

Es hat sich gezeigt, daß der Einsatz bestimmter Polymere die vorstehend genannten Aufgaben sowohl in wäßrigen als auch in wasserfreien Systemen löst.

Gegenstand der vorliegenden Erfindung sind in einer ersten Ausführungsform Mittel zum Aufhellen keratinischer Fasern, enthaltend - bezogen auf ihr Gewicht -
a) 0,01 bis 5 Gew.-% Ethylcellulose,
b) 5 bis 70 Gew.-% Ölkomponente(n),
c) 1 bis 70 Gew.-% Peroxidisulfat(e) aus der Gruppe Natriumperoxodisulfat und/oder Kaliumperoxodisulfat und/oder Ammoniumperoxodisulfat.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Als ersten Inhaltsstoff enthalten die erfindungsgemäßen Mittel Ethylcellulose.

Besonders bevorzugte erfindungsgemäße Mittel sind dadurch gekennzeichnet, daß sie 0,15 bis 5 Gew.-%, besonders bevorzugt 0,2 bis 4 Gew.-% und insbesondere 0,3 bis 1,5 Gew.-% Ethylcellulose der Formel (I) 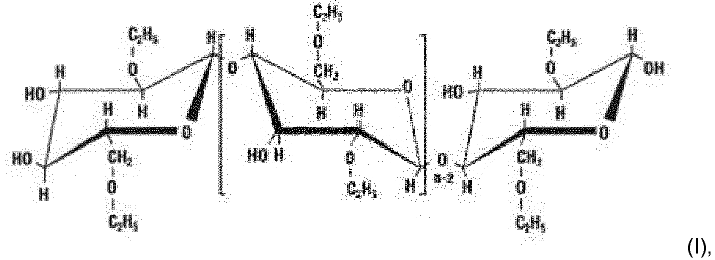 worin n für ganze Zahlen von 50 bis 500, vorzugsweise von 100 bis 400, weiter bevorzugt von 150 bis 300 und insbesondere von 200 bis 250 steht.

Der Substitutionsgrad, d.h. die durchschnittliche Zahl veretherter Hydroxygruppen pro Glucoseeinheit" liegt für die in den erfindungsgemäßen Mitteln eingesetzte(n) Ethylcellulose(n) vorzugsweise zwischen 20 und 60 %. Besonders bevorzugte erfindungsgemäße Mittel sind dadurch gekennzeichnet, daß sie 0,15 bis 5 Gew.-%, besonders bevorzugt 0,2 bis 4 Gew.-% und insbesondere 0,3 bis 1,5 Gew.-% Ethylcellulose(n) enthalten, deren Substitutionsgrad 45 bis 50 %, vorzugsweise 46 bis 49,9%, weiter bevorzugt 47 bis 49,8%, noch weiter bevorzugt 47,5 bis 49,7% und insbesondere 48 bis 49,5% beträgt.

Die Ethylcellulose(n) führen in Kombination mit den Inhaltsstoffen b) und c) zu angenehm viskosen Blondierpasten, die eine hervorragende Stabilität bei drastisch verringerter Ölabscheidung zeigen. Zudem ist die Mischbarkeit mit farbstoffhaltigen Formulierungen und mit der Entwickleremulsion deutlich verbessert. Auch die fertig angemischten Blondiermittel zeigen eine für das Anwendungsgebiet sehr geeignete und stabile Viskosität.

Als zweiten Inhaltsstoff enthalten die erfindungsgemäßen Mittel 5 bis 70 Gew.-% Ölkomponente(n). Vorzugsweise. ist/sind diese(s) Öl(e) bei 25°C flüssig.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.

Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Ein erfindungsgemäß bevorzugter Cyclomethicone-Ersatzstoff ist eine Mischung aus C₁₃-C₁₆-Iso-paraffinen, C₁₂ - C₁₄-Isoparaffinen und C₁₃ - C₁₅-Alkanen, deren Viskosität bei 25°C im Bereich von 2 bis 6 mPas liegt und die einen Dampfdruck bei 20°C im Bereich von 10 bis 150 Pa, bevorzugt 100 bis 150 Pa, aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning® 200 (0,65 cSt) und Dow Corning® 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Weitere bevorzugte erfindungsgemäße Produkte enthalten mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa.

Als kosmetisches Öl erfindungsgemäß weiterhin besonders bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat, Ethylenglycoldipalmitat. n-Hexyllaurat, n-Decyloleat, Oleyloleat, Oleylerucat, Erucyloleat, C₁₂-C₁₅-Alkyllactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv® TN (C₁₂-C₁₅-Alkyl-benzoat), sowie Benzoesäureisostearylester, z. B. erhältlich als Finsolv® SB, 2-Ethylhexylbenzoat, z. B. erhältlich als Finsolv® EB, und Benzoesäure-2-octyldodecylester, z. B. erhältlich als Finsolv® BOD.

Als besonders vorteilhaft hat sich der Einsatz von Isopropylestern von C₁₂-C₁₈-Carbonsäuren, insbesondere der Einsatz von Isopropylmyristat, und besonders bevorzugt Mischungen von Isopropylmyristat mit C₁₀-C₁₃-Isoparaffin-Mischungen, letztere bevorzugt mit einem Dampfdruck bei 20°C von 10 - 400 Pa, erwiesen.

Ein weiteres besonders bevorzugtes Esteröl ist Triethylcitrat. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine. Weitere erfindungsgemäß bevorzugte Produkte enthalten Triethylcitrat und eine Mischung aus Isodecan, Isoundecan, Isododecan und Isotridecan.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol® 318 oder Myritol® 331 (BASF/ Cognis) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in dem erfindungsgemäßen Produkt aus.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol® CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Besonders bevorzugt beträgt das Gesamtgewicht an Dimerfettsäureestern 0,5 - 10 Gew.-%, bevorzugt 1 - 5 Gew.-%, jeweils bezogen auf das Gewicht der gesamten Wasser-in-ÖI-Emulsion, ohne das Gewicht des Treibmittels zu berücksichtigen.

Weitere kosmetische Öle, die erfindungsgemäß besonders bevorzugt sind, sind ausgewählt aus nichtflüchtigen Siliconölen. Erfindungsgemäß bevorzugte nichtflüchtige Siliconöle sind ausgewählt aus linearen Polyalkylsiloxanen mit einer kinematischen Viskosität bei 25°C von mindestens 5 cSt bis 2000 cSt, insbesondere ausgewählt aus linearen Polydimethylsiloxanen mit einer kinematischen Viskosität bei 25°C von 5 cSt bis 2000 cSt, bevorzugt 10 bis 350 cSt, besonders bevorzugt 50 - 100 cSt, wie sie z. B. unter den Handelsnamen Dow Corning® 200 bzw. Xiameter PMX von Dow Corning bzw. Xiameter erhältlich sind. Weitere bevorzugte nichtflüchtige Siliconöle sind Phenyltrimethicone mit einer kinematischen Viskosität bei 25°C von 10 bis 100 cSt, bevorzugt von 15 - 30 cSt sowie Cetyldimethicone.

Erfindungsgemäß bevorzugte Mittel enthalten mindestens ein nichtflüchtiges Siliconöl, das bevorzugt ausgewählt ist aus linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 0,1 - 30 Gew.-%, bevorzugt 1 - 24 Gew.-%, besonders bevorzugt 2 - 18 Gew.-%, außerordentlich bevorzugt 4 - 10 Gew.-%, jeweils bezogen auf das Gewicht des gesamten Mittels.

Von den genannten Ölen haben sich einige als besonders geeignet erwiesen, da sie die physikalische und chemische Stabilität der Blondiermittelpasten über lange Zeiträume garantieren und mit den weiteren erfindungsgemäßen Inhaltsstoffen hervorragend verträglich sind. Erfindungsgemäß bevorzugte Mittel sind dadurch gekennzeichnet, daß sie 22,5 bis 70 Gew.-%, vorzugsweise 25 bis 65 Gew.-%, weiter bevorzugt 27,5 bis 60 Gew.-%, besonders bevorzugt 30 bis 55 Gew.-% und insbesondere 32,5 bis 50 Gew.-% Öl(e) aus der Gruppe Paraffinöl, Polyisobuten, der Alkylbenzoate, Isopropylpalmitat, der C₁₄₋₂₂-Alkane, Isononyl-Isononanoat enthalten.

Als weiteren wesentlichen Inhaltsstoff enthalten die erfindungsgemäßen Zubereitungen 1 bis 70 Gew.-% Peroxidisulfat(e) aus der Gruppe Natriumperoxodisulfat und/oder Kaliumperoxodisulfat und/oder Ammoniumperoxodisulfat,

Hierbei haben sich Mittel als besonders geeignet erweisen, welche bestimmte Peroxodisulfate in engeren Mengenbereichen enthalten. Äußerst bevorzugte Mittel enthalten 2,5 bis 65 Gew.-%, vorzugsweise 5 bis 60 Gew.-%, weiter bevorzugt 7,5 bis 55 Gew.-%, besonders bevorzugt 10 bis 50 Gew.-% und insbesondere 12,5 bis 45 Gew.-% Peroxidisulfat(e) aus der Gruppe Natriumperoxodisulfat und/oder Kaliumperoxidisulfat.

Es ist äußerst bevorzugt, die Menge an Kaliumperoxidisulfat immer deutlich größer zu halten als die Menge an eventuell eingesetztem Natrium- und Ammoniumperoxidisulfat. Es hat sich gezeigt, daß mit steigendem Kaliumperoxidisulfat-Anteil an der Gesamtmenge an Peroxidisulfaten die chemische und physikalische Stabilität der erfindungsgemäßen Mittel steigt. In bevorzugten Mittel liegt daher das Gewichtsverhältnis von Kaliumperoxidisulfat zu Natrium- und Ammoniumperoxidisulfat bei > 2, vorzugsweise bei > 5, weiter bevorzugt bei > 10, noch weiter bevorzugt bei > 15 und insbesondere bei > 20. Dieses Gewichtsverhältnis wird ermittelt, indem die Gew.-%-Menge an Kaliumperoxidisulfat durch die Summe der Gew.-%-Mengen an Natrium- und Ammoniumperoxidisulfat dividiert wird.

Erfindungsgemäß bevorzugte Mittel sind dadurch gekennzeichnet, daß das Gewichtsverhältnis von im Mittel enthaltenem Kaliumperoxidisulfat zu im Mittel enthaltenen Natrium- und Ammonium-Peroxidisulfaten > 10:1, vorzugsweise > 12,5 :1, weiter bevorzugt > 15:1, besonders bevorzugt > 17,5:1 und insbesondere > 20:1 beträgt.

Äußerst bevorzugte erfindungsgemäße Mittel enthalten 0 bis < 2,5 Gew.-%, vorzugsweise 0 bis < 1 Gew.-%, weiter bevorzugt 0 bis < 0,5 Gew.-%, besonders bevorzugt 0 bis < 0,1 Gew.-% und insbesondere 0 Gew.-% Peroxidisulfate aus der Gruppe Natriumperoxidisulfat und/oder Ammoniumperoxidisulfat.

Als weiteren Inhaltstoff können die erfindungsgemäßen Mittel mindestens ein natürliches Polymer enthalten. Als natürliches Polymer können beispielsweise Cellulosederivate verwendet werden, die als Verdickungsmittel Verwendung finden. Beispiele sind Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Staerke-Fraktionen und Derivate wie Amylose, Amylopektin und Dextrine, Gelatine und Casein sowie Cellulosederivate, wie beispielsweise Methylcellulose, Carboxyalkylcellulosen wie Carboxymethylcellulose und Hydroxyalkylcellulosen wie Hydroxyethylcellulose.

Natürliche Polymere aus den genannten Substanzklassen sind kommerziell erhältlich und werden beispielsweise unter den Handelsnamen Deuteron®-XG (anionisches Heteropolysaccharid auf Basis von β-D-Glucose, D-Manose, D-Glucuronsaeure, Schoener GmbH), Deuteron®-XN (nichtionogenes Polysaccharid, Schoener GmbH), Protanal RF 6650 alginate (Natriumalginat, FMC Biopolymer), Cekol (Cellulose Gum, Kelco), Kelzan (Xanthan-Biopolymer, Kelco), Xanthan FN (Xanthan-Biopolymer, Jungbunzlauer), Keltrol z.B. Keltrol CG-T (Xanthan-Biopolymer, Kelco) oder Keltrol CG-SFT (Xanthan-Biopolymer, Kelco) angeboten.

In einer bevorzugten Ausführungsform der Erfindung enthalten die erfindungsgemäßen Mittel Xanthan. Erfindungsgemäß bevorzugt sind solche Xanthane, die nach der Quellung transparente Zubereitungen ergeben. Insbesondere bevorzugt ist die Verwendung des Xanthan-Biopolymers, welches unter dem Handelsnamen Keltrol CG-SFT der Firma Kelco vertrieben wird.

In einer bevorzugten Ausführungsform enthält ein erfindungsgemäßes Mittel 0,1 bis 5 Gew.-%, vorzugsweise 0,5 bis 4 Gew.-%, weiter bevorzugt 1 bis 3 Gew.-%, besonders bevorzugt 1,25 bis 2,5 Gew.-% und insbesondere 1,5 bis 2 Gew.-% Xanthan.

Als Konsistenzgeber können die erfindungsgemäßen Mittel bevorzugt langkettige Fettalkohole enthalten, die vorzugsweise aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13*E*)-Docosen-1-ol) ausgewählt sind.

Diese langkettigen Fettalkohole besitzen ein Kettenlänge von mindestens 20 C-Atomen. Innerhalb dieser Gruppe haben sich spezielle langkettige Fettalkohole als ganz besonders geeignet erwiesen. In einer besonders bevorzugten Ausführungsform ist ein Mittel zum Blondieren und/oder Bleichen von keratinischen Fasern dadurch gekennzeichnet, daß es Arachylalkohol (Eicosan-1-ol) enthält.

In einer weiteren besonders bevorzugten Ausführungsform ist ein Mittel zum Blondieren und/oder Bleichen von keratinischen Fasern dadurch gekennzeichnet, daß es Arachylalkohol (Eicosan-1-ol) und/oder Behenylalkohol (Docosan-1-ol) enthält.

Weiterhin hat sich herausgestellt, daß es von Vorteil ist, wenn die langkettigen Fettalkohole, insbesondere Arachylalkohol (Eicosan-1-ol) und/oder Behenylalkohol (Docosan-1-ol), in bestimmten Mengenbereichen in dem erfindungsgemäßen Mittel enthalten sind. Bevorzugte erfindungsgemäße Mittel enthalten ein oder mehrere langkettige Fettalkohole (a) aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels.

In einer ganz besonders bevorzugten Ausführungsform ist ein erfindungsgemäßes Mittel dadurch gekennzeichnet, daß es als Fettalkohol(e) Arachylalkohol (Eicosan-1-ol) und/oder Behenylalkohol (Docosan-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels - enthält.

Neben den speziellen langkettigen Fettalkoholen mit einer Kettenlänge von mindestens 20 C-Atomen kann das erfindungsgemäße Mittel zusätzlich auch noch weitere, kürzerkettige Fettalkohole mit einer Kettenlänge von 12 bis 18 C-Atomen enthalten. Geeignete kürzerkettige Fettalkohole mit einer gesättigten C₁₂-C₁₈-Alkylkette sind beispielsweise Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol) und Octadecan-1-ol (Octadecylalkohol, Stearylalkohol). Ein geeigneter kürzerkettiger Fettalkohol mit einer ungesättigten C₁₂-C₁₈-Alkylkette ist beispielsweise (9*Z*)-Octadec-9-en-1-ol (Oleylalkohol).

Zur physikalischen und chemischen Stabilisierung besonders geeignet ist Cetylstearylakohol. Ganz besonders bevorzugte erfindungsgemäße Mittel enthalten 1 bis 15 Gew.-%, vorzugsweise 2 bis 12,5 Gew.-%, weiter bevorzugt 3,5 bis 11 Gew.-%, besonders bevorzugt 4 bis 10 Gew.-% und insbesondere 5 bis 7,5 Gew.-% Cetearlyalkohol.

Weiterhin können die Blondiermittel Alkalisierungsmittel enthalten. Bevorzugte Alkalisierungsmittel sind beispielsweise Ammoniak, Alkanolamine, basischen Aminosäuren, sowie anorganischen Alkalisierungsmittel wie (Erd-)Alkalimetallhydroxide, (Erd-)Alkalimetallmetasilikate, (Erd-) Alkalimetallphosphate und (Erd-)Alkalimetallhydrogenphosphate. Als Metallionen dienen bevorzugt Lithium, Natrium und/oder Kalium.

Erfindungsgemäß einsetzbare, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Silicate.

Erfindungsgemäß einsetzbare Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Amino-propan-1,2-diol, 2-Amino-2-methylpropan-1,3-diol, 2-Amino-2-ethyl-1,3-propandiol, N,N-Dimethylethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und/oder D/L-Histidin. Besonders bevorzugt werden L-Arginin, D-Arginin und/oder D/L-Arginin als ein Alkalisierungsmittel im Sinne der Erfindung eingesetzt.

Manche Kunden empfinden die intensive Geruchsbildung von Ammoniak belästigend oder störend. Obwohl Ammoniak ein bevorzugtes Alkalisierungsmittel ist, können daher anwendungsbereite Zubereitungen erfindungsgemäß bevorzugt sein, die frei von Ammoniak sind. Bevorzugte Alkalisierungsmittel für Zubereitungen, die frei von Ammoniak sind, sind Monoethanolamin, 2-Amino-2-methyl-propanol und Triethanolamin.

Erfindungsgemäß besonders bevorzugte Mittel sind dadurch gekennzeichnet, daß sie 0 bis < 0,1 Gew.-%, vorzugsweise 0 bis < 0,05 Gew.-%, weiter bevorzugt 0 bis < 0,01 Gew.-%, besonders bevorzugt 0 bis < 0,001 Gew.-% und insbesondere 0 Gew.-% Ammoniak enthalten.

Wenn die anwendungsbereiten Mischungen Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Die erfindungsgemäßen Zusammensetzungen können zusätzlich mindestens einen weiteren Bleichverstärker, der von den anorganischen Persalzen verschieden ist, enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Die erfindungsgemäßen Zusammensetzungen sind bereits kurz nach ihrer Herstellung viskositäts- und absetzstabil. Vorzugsweise werden die Mengen der Inhaltsstoffe so aufeinander abgestimmt, daß die fertigen Mittel im Anschluß an die Herstellung nach kurzer Ruhezeit eine für die Produktkategorie geeignete Viskosität aufweisen. Besonders bevorzugte Mittel sind dabei dadurch gekennzeichnet, daß sie 24 h nach der Herstellung bei 25°C eine Viskosität (Brookfield RV-2, Helipath, Spindel TF, 4 U/min, 60 s) von 100 Pas bis 10.000 Pas (10⁵ mPas bis 10⁷ mPas), vorzugsweise von 150 Pas bis 7.500 Pas (1,5 x 10⁵ mPas bis 7,5 x 10⁶ mPas), weiter bevorzugt von 200 Pas bis 5.000 Pas (2 x 10⁵ mPas bis 5 x 10⁶ mPas) und insbesondere von 250 Pas bis 2.000 Pas (2,5 x 10⁵ mPas bis 2 x 10⁶ mPas) aufweisen.

Ein zweiter Gegenstand der Erfindung ist ein Verfahren zur Farbveränderung keratinischer Fasern, bei dem mindestens zwei getrennt voneinander verpackte Zubereitungen (A) und (B), von welchen Zubereitung (A) mindestens ein Persulfat und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, dadurch gekennzeichnet, daß Zubereitung (A)
a) 0,01 bis 5 Gew.-% Ethylcellulose,
b) 5 bis 70 Gew.-% Ölkomponente(n),
c) 1 bis 70 Gew.-% Peroxidisulfat(e) aus der Gruppe Natriumperoxodisulfat und/oder Kaliumperoxodisulfat
enthält.

Die anwendungsbereiten Mittel werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Die erfindungsgemäßen Mittel werden in einem Verfahren zum Aufhellen von keratinischen Fasern, insbesondere menschlichen Haaren, angewendet, bei dem das Mittel auf die keratinhaltigen Fasern aufgebracht, bei einer Temperatur von Raumtemperatur bis 45 °C für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Bevorzugt liegt die Temperatur während der Einwirkzeit zwischen 20°C und 40°C, insbesondere zwischen 25°C und 38°C. Die Aufhellmittel ergeben bereits bei physiologisch verträglichen Temperaturen von unter 45°C gute Blondier- und Aufhellergebnisse.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensid-haltigen Träger besitzt.

Die bevorzugten Ausführungsformen des ersten Erfindungsgegenstands gelten mutatis mutandis auch für den zweiten Erfindungsgegenstand.

Hinsichtlich der Viskosität der Anwendungsmischung aus dem erfindungsgemäßen Mittel und der Entwickleremulson bzw. weiteren Komponenten sind erfindungsgemäße Verfahren bevorzugt, bei denen die Anwendungsmischung 24 h nach der Herstellung bei 25°C eine Viskosität (Brookfield RV-2, Helipath, Spindel TF, 4 U/min, 60 s) von 1 Pas bis 100 Pas (10³ mPas bis 10⁵ mPas), vorzugsweise von 5 Pas bis 80 Pas (5 x 10³ mPas bis 8 x 10⁴ mPas), weiter bevorzugt von 10 Pas bis 65 Pas (10⁴ mPas bis 6,5 x 10⁴ mPas) und insbesondere von 10 Pas bis 50 Pas (10⁴ mPas bis 5 x 10⁴ mPas) aufweist.

## Patentansprüche

1. Mittel zum Aufhellen keratinischer Fasern, enthaltend - bezogen auf sein Gewicht -
a) 0,01 bis 5 Gew.-% Ethylcellulose,
b) 5 bis 70 Gew.-% Ölkomponente(n),
c) 1 bis 70 Gew.-% Peroxidisulfat(e) aus der Gruppe Natriumperoxodisulfat und/oder Kaliumperoxodisulfat und/oder Ammoniumperoxodisulfat.

2. Mittel nach Anspruch 1, **dadurch gekennzeichnet, daß** es 0,15 bis 5 Gew.-%, besonders bevorzugt 0,2 bis 4 Gew.-% und insbesondere 0,3 bis 1,5 Gew.-% Ethylcellulose der Formel (I) 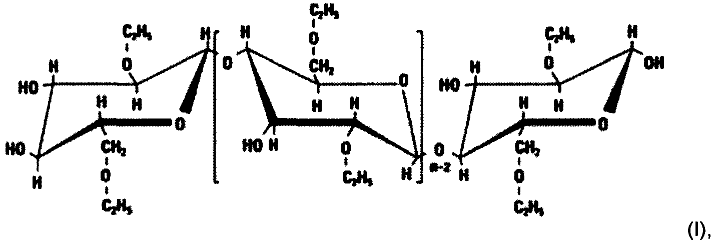 worin n für ganze Zahlen von 50 bis 500, vorzugsweise von 100 bis 400, weiter bevorzugt von 150 bis 300 und insbesondere von 200 bis 250 steht.

3. Mittel nach einem der Ansprüche 1 oder 2, **dadurch gekennzeichnet, daß** es 0,15 bis 5 Gew.-%, besonders bevorzugt 0,2 bis 4 Gew.-% und insbesondere 0,3 bis 1,5 Gew.-% Ethylcellulose(n) enthält, deren Ethoxylierungsgrad 45 bis 50 %, vorzugsweise 46 bis 49,9%, weiter bevorzugt 47 bis 49,8%, noch weiter bevorzugt 47,5 bis 49,7% und insbesondere 48 bis 49,5% beträgt.

4. Mittel nach einem der Ansprüche 1 bis 3, **dadurch gekennzeichnet, daß** es 22,5 bis 70 Gew.-%, vorzugsweise 25 bis 65 Gew.-%, weiter bevorzugt 27,5 bis 60 Gew.-%, besonders bevorzugt 30 bis 55 Gew.-% und insbesondere 32,5 bis 50 Gew.-% Öl(e) aus der Gruppe Paraffinöl, Polyisobuten, der Alkylbenzoate, Isopropylpalmitat, Isohexadecan, Isododecan, Isononyl-Isononanoat enthält.

5. Mittel nach einem der Ansprüche 1 bis 4, **dadurch gekennzeichnet, daß** es 1 bis 15 Gew.-%, vorzugsweise 2 bis 12,5 Gew.-%, weiter bevorzugt 3,5 bis 11 Gew.-%, besonders bevorzugt 4 bis 10 Gew.-% und insbesondere 5 bis 7,5 Gew.-% Cetearlyalkohol enthält.

6. Mittel nach einem der Ansprüche 1 bis 5, **dadurch gekennzeichnet, daß** es 2,5 bis 65 Gew.-%, vorzugsweise 5 bis 60 Gew.-%, weiter bevorzugt 7,5 bis 55 Gew.-%, besonders bevorzugt 10 bis 50 Gew.-% und insbesondere 12,5 bis 45 Gew.-% Peroxidisulfat(e) aus der Gruppe Natriumperoxodisulfat und/oder Kaliumperoxidisulfat enthält.

7. Mittel nach einem der Ansprüche 1 bis 6, **dadurch gekennzeichnet, daß** es 0 bis < 0,1 Gew.-%, vorzugsweise 0 bis < 0,05 Gew.-%, weiter bevorzugt 0 bis < 0,01 Gew.-%, besonders bevorzugt 0 bis < 0,001 Gew.-% und insbesondere 0 Gew.-% Ammoniak enthält.

8. Mittel nach einem der Ansprüche 1 bis 7, **dadurch gekennzeichnet, daß** es bei 25°C eine Viskosität (Brookfield RV-2, Helipath, Spindel TF, 4 U/min, 60 s) von 100 Pas bis 10.000 Pas (10⁵ mPas bis 10⁷ mPas), vorzugsweise von 150 Pas bis 7.500 Pas (1,5 x 10⁵ mPas bis 7,5 x 10⁶ mPas), weiter bevorzugt von 200 Pas bis 5.000 Pas (2 x 10⁵ mPas bis 5 x 10⁶ mPas) und insbesondere von 250 Pas bis 2.000 Pas (2,5 x 10⁵ mPas bis 2 x 10⁶ mPas) aufweist.

9. Verfahren zur Farbveränderung keratinischer Fasern, bei dem mindestens zwei getrennt voneinander verpackte Zubereitungen (A) und (B), von welchen Zubereitung (A) mindestens ein Persulfat und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, **dadurch gekennzeichnet, daß** Zubereitung (A)
a) 0,01 bis 5 Gew.-% Ethylcellulose,
b) 5 bis 70 Gew.-% Ölkomponente(n),
c) 1 bis 70 Gew.-% Peroxidisulfat(e) aus der Gruppe Natriumperoxodisulfat und/oder Kaliumperoxodisulfat
enthält.

10. Verfahren nach Anspruch 9, **dadurch gekennzeichnet, daß** die Anwendungsmischung bei 25°C eine Viskosität (Brookfield RV-2, Helipath, Spindel TF, 4 U/min, 60 s) von 1 Pas bis 100 Pas (10³ mPas bis 10⁵ mPas), vorzugsweise von 5 Pas bis 80 Pas (5 x 10³ mPas bis 8 x 10⁴ mPas), weiter bevorzugt von 10 Pas bis 65 Pas (10⁴ mPas bis 6,5 x 10⁴ mPas) und insbesondere von 10 Pas bis 50 Pas (10⁴ mPas bis 5 x 10⁴ mPas) aufweist.

## Claims

1. An agent for lightening keratin fibers, containing, based on its weight,
a) 0.01 to 5 wt.% ethyl cellulose,
b) 5 to 70 wt.% oil component(s),
c) 1 to 70 wt.% peroxydisulfate(s) from the group of sodium peroxodisulfate and/or potassium peroxodisulfate and/or ammonium peroxodisulfate.

2. The agent according to claim 1, **characterized in that** it contains 0.15 to 5 wt.%, particularly preferably 0.2 to 4 wt.%, and in particular 0.3 to 1.5 wt.% ethyl cellulose of formula (I) 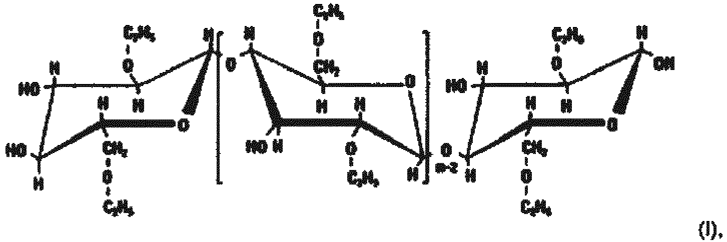 where n stands for integers from 50 to 500, preferably from 100 to 400, more preferably from 150 to 300 and in particular from 200 to 250.

3. The agent according to one of claims 1 or 2, **characterized in that** it contains 0.15 to 5 wt.%, particularly preferably 0.2 to 4 wt.% and in particular 0.3 to 1.5 wt.% ethyl cellulose(s) of which the degree of ethoxylation is 45 to 50%, preferably 46 to 49.9%, more preferably 47 to 49.8%, even more preferably 47.5 to 49.7% and in particular 48 to 49.5%.

4. The agent according to one of claims 1 to 3, **characterized in that** it contains 22.5 to 70 wt.%, preferably 25 to 65 wt.%, more preferably 27.5 to 60 wt.%, particularly preferably 30 to 55 wt.%, and in particular 32.5 to 50 wt.% oil(s) from the group of paraffin oil, polyisobutene, the alkyl benzoates, isopropyl palmitate, isohexadecane, isododecane and isononyl isononanoate.

5. The agent according to one of claims 1 to 4, **characterized in that** it contains 1 to 15 wt.%, preferably 2 to 12.5 wt.%, more preferably 3.5 to 11 wt.%, particularly preferably 4 to 10 wt.% and in particular 5 to 7.5 wt.% cetearyl alcohol.

6. The agent according to one of claims 1 to 5, **characterized in that** it contains 2.5 to 65 wt.%, preferably 5 to 60 wt.%, more preferably 7.5 to 55 wt.%, particularly preferably 10 to 50 wt.%, and in particular 12.5 to 45 wt.% peroxydisulfate(s) from the group of sodium peroxodisulfate and/or potassium peroxydisulfate.

7. The agent according to one of claims 1 to 6, **characterized in that** it contains 0 to < 0.1 wt.%, preferably 0 to < 0.05 wt.%, more preferably 0 to < 0.01 wt.%, particularly preferably 0 to < 0.001 wt.%, and in particular 0 wt.% ammonia.

8. The agent according to one of claims 1 to 7, **characterized in that**, at 25 °C, it has a viscosity (Brookfield RV-2, Helipath, spindle TF, 4 rpm, 60 s) of from 100 Pas to 10,000 Pas (10⁵ mPas to 10⁷ mPas), preferably from 150 Pas to 7,500 Pas (1.5 × 10⁵ mPas to 7.5 × 10⁶ mPas), more preferably from 200 Pas to 5,000 Pas (2 × 10⁵ mPas to 5 × 10⁶ mPas), and in particular from 250 Pas to 2,000 Pas (2.5 × 10⁵ mPas to 2 × 10⁶ mPas).

9. A method for changing the color of keratin fibers, in which at least two separately packaged preparations (A) and (B), of which preparation (A) contains at least one persulfate and preparation (B) contains at least one oxidizing agent, are mixed to form an application mixture, said mixture is applied to the fibers and is rinsed off again after a contact time, **characterized in that** preparation (A) contains
a) 0.01 to 5 wt.% ethyl cellulose,
b) 5 to 70 wt.% oil component(s),
c) 1 to 70 wt.% peroxydisulfate(s) from the group of sodium peroxodisulfate and/or potassium peroxodisulfate.

10. The method according to claim 9, **characterized in that**, at 25 °C, the application mixture has a viscosity (Brookfield RV-2, Helipath, spindle TF, 4 rpm, 60 s) of from 1 Pas to 100 Pas (10³ mPas to 10⁵ mPas), preferably from 5 Pas to 80 Pas (5 × 10³ mPas to 8 × 10⁴ mPas), more preferably from 10 Pas to 65 Pas (10⁴ mPas to 6.5 × 10⁴ mPas), and in particular from 10 Pas to 50 Pas (10⁴ mPas to 5 × 10⁴ mPas).

## Revendications

1. Agent pour l'éclaircissement des fibres kératiniques, contenant - rapporté à son poids -
a) de 0,01 à 5 % en poids d'éthylcellulose,
b) de 5 à 70 % en poids de composant(s) d'huile,
c) de 1 à 70 % en poids de disulfate(s) de peroxyde du groupe constitué par le peroxodisulfate de sodium et/ou le peroxodisulfate de potassium et/ou le peroxodisulfate d'ammonium.

2. Agent selon la revendication 1, **caractérisé en ce que** qu'il contient de 0,15 à 5 % en poids, de manière particulièrement préférée de 0,2 à 4 % en poids et en particulier de 0,3 à 1,5 % en poids d'éthylcellulose de la formule (I) 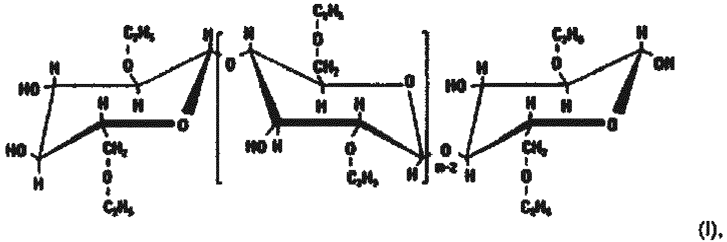 dans laquelle n représente des nombres entiers allant de 50 à 500, de préférence de 100 à 400, de manière davantage préférée de 150 à 300 et notamment de 200 à 250.

3. Agent selon l'une quelconque des revendications 1 ou 2, **caractérisé en ce qu'**il contient de 0,15 à 5 % en poids, de manière particulièrement préférée de 0,2 à 4 % en poids et notamment de 0,3 à 1,5 % en poids d'éthylcellulose(s), dont le degré d'éthoxylation vaut de 45 à 50 %, de préférence de 46 à 49,9 %, de manière davantage préférée de 47 à 49,8 %, de manière encore davantage préférée de 47,5 à 49,7 % et notamment de 48 à 49,5 %.

4. Agent selon l'une quelconque des revendications 1 à 3, **caractérisé en ce qu'**il contient de 22,5 à 70 % en poids, de préférence de 25 à 65 % en poids, de manière davantage préférée de 27,5 à 60 % en poids, de manière particulièrement préférée de 30 à 55 % en poids et notamment de 32,5 à 50 % en poids en huile(s) issue(s) du groupe des huiles de paraffine, des polyisobutènes, des benzoates d'alkyle, du palmitate d'isopropyle, de l'isohexadécane, de l'isododécane, de l'isononanoate d'isononyle.

5. Agent selon l'une quelconque des revendications 1 à 4, **caractérisé en ce qu'**il contient de 1 à 15 % en poids, de préférence de 2 à 12,5 % en poids, de manière davantage préférée de 3,5 à 11 % en poids, de manière particulièrement préférée de 4 à 10 % en poids et notamment de 5 à 7,5 % en poids d'alcool cétéarylique.

6. Agent selon l'une quelconque des revendications 1 à 5, **caractérisé en ce qu'**il contient de 2,5 à 65 % en poids, de préférence de 5 à 60 % en poids, de manière davantage préférée de 7,5 à 55 % en poids, de manière particulièrement préférée de 10 à 50 % en poids et notamment de 12,5 à 45 % en poids en disulfate(s) de peroxyde issus du groupe du peroxodisulfate de sodium et/ou du peroxodisulfate de potassium.

7. Agent selon l'une quelconque des revendications 1 à 6, **caractérisé en ce qu'**il contient de 0 à < 0,1 % en poids, de préférence de 0 à < 0,05 % en poids, de manière davantage préférée de 0 à < 0,01 % en poids, de manière particulièrement préférée de 0 à < 0,001 % en poids et notamment 0 % en poids d'ammoniaque.

8. Agent selon l'une quelconque des revendications 1 à 7, **caractérisé en ce qu'**il présente à 25 °C une viscosité (Brookfield RV-2, Helipath, broche TF, 4 t/min, 60 s) de 100 Pas à 10 000 Pas (10⁵ mPas à 10⁷ mPas), de préférence de 150 Pas à 7 500 Pas (1,5 x 10⁵ mPas à 7,5 x 10⁶ mPas), de manière davantage préférée de 200 Pas à 5 000 Pas (2 x 10⁵ mPas à 5 x 10⁶ mPas) et notamment de 250 Pas à 2 000 Pas (2,5 x 10⁵ mPas à 2 x 10⁶ mPas).

9. Procédé de modification de la couleur de fibres kératiniques, dans lequel on mélange au moins deux préparations (A) et (B) conditionnées séparément l'une de l'autre, dont la préparation (A) contient au moins un persulfate et la préparation (B) contient au moins un agent oxydant, pour former un mélange d'application, qui est appliqué sur les fibres et qui est rincé après un temps d'action, **caractérisé en ce que** la préparation (A) contient
a) de 0,01 à 5 % en poids d'éthylcellulose,
b) de 5 à 70 % en poids de composant(s) d'huile,
c) de 1 à 70 % en poids en disulfate(s) de peroxyde issus du groupe du peroxodisulfate de sodium et/ou du peroxodisulfate de potassium.

10. Procédé selon la revendication 9, **caractérisé en ce que** le mélange d'application présente à 25 °C une viscosité (Brookfield RV-2, Helipath, broche TF, 4 t/min, 60 s) de 1 Pas à 100 Pas (10³ mPas à 10⁵ mPas), de préférence de 5 Pas à 80 Pas (5 x 10³ mPas à 8 x 10⁴ mPas), de manière davantage préférée de 10 Pas à 65 Pas (10⁴ mPas à 6,5 x 10⁴mPas) et notamment de 10 Pas à 50 Pas (10⁴mPas à 5 x 10⁴ mPas).
